(19) Europäisches Patentamt / European Patent Office / Office européen des brevets

(11) **EP 4 168 769 B1**

(12) **EUROPÄISCHE PATENTSCHRIFT**

(45) Veröffentlichungstag und Bekanntmachung des Hinweises auf die Patenterteilung:
**10.04.2024 Patentblatt 2024/15**

(21) Anmeldenummer: **21737554.2**

(22) Anmeldetag: **16.06.2021**

(51) Internationale Patentklassifikation (IPC):
**G01N 1/40** *(2006.01)*

(52) Gemeinsame Patentklassifikation (CPC):
**G01N 1/40; B03C 7/006; B03C 7/06;
G01N 15/0266; G01N 15/10; G01N 25/005;
G01N 25/20; G01N 33/442;** G01N 2001/4038;
G01N 2015/0288; G01N 2015/1028

(86) Internationale Anmeldenummer:
**PCT/DE2021/100516**

(87) Internationale Veröffentlichungsnummer:
**WO 2021/254566 (23.12.2021 Gazette 2021/51)**

(54) **VERFAHREN ZUR QUANTIFIZIERUNG VON POLYMERSPEZIES IN EINER POLYMERPARTIKEL ENTHALTENDEN PROBE**

METHOD FOR QUANTIFYING POLYMER SPECIES IN A SPECIMEN CONTAINING POLYMER PARTICLES

PROCÉDÉ DE QUANTIFICATION D'ESPÈCES POLYMÈRES DANS UN ÉCHANTILLON CONTENANT DES PARTICULES POLYMÈRES

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **17.06.2020 DE 102020115971**

(43) Veröffentlichungstag der Anmeldung:
**26.04.2023 Patentblatt 2023/17**

(73) Patentinhaber: **Hochschule für Technik und Wirtschaft Dresden
01069 Dresden (DE)**

(72) Erfinder:
- **HARRE, Kathrin
  01109 Dresden (DE)**
- **GROSSMANN, Carsten
  01157 Dresden (DE)**
- **WEISE, Mandy
  01127 Dresden (DE)**
- **SOCHER, Martin
  01187 Dresden (DE)**
- **GRISCHEK, Thomas
  01156 Dresden (DE)**
- **BAUER, Reinhard
  01705 Freital (DE)**
- **SIMON, Frank
  01099 Dresden (DE)**

(74) Vertreter: **Sperling, Thomas
  Sperling, Fischer & Heyner
  Patentanwälte
  Tolkewitzer Straße 22
  01277 Dresden (DE)**

(56) Entgegenhaltungen:
**US-A1- 2003 226 917    US-B1- 6 681 938**

- **CUTRONEO LAURA ET AL: "Microplastics in seawater: sampling strategies, laboratory methodologies, and identification techniques applied to port environment", ENVIRONMENTAL SCIENCE AND POLLUTION RESEARCH, ECOMED, LANDSBERG, DE, Bd. 27, Nr. 9, 6. Februar 2020 (2020-02-06), Seiten 8938-8952, XP037128267, ISSN: 0944-1344, DOI: 10.1007/S11356-020-07783-8 [gefunden am 2020-02-06]**

- ZHANG BIN ET AL: "Research Progress of Microplastics in Freshwater Sediments in China", ENVIRONMENTAL SCIENCE AND POLLUTION RESEARCH, ECOMED, LANDSBERG, DE, Bd. 27, Nr. 25, 8. Juni 2020 (2020-06-08), Seiten 31046-31060, XP037206868, ISSN: 0944-1344, DOI: 10.1007/S11356-020-09473-X [gefunden am 2020-06-08]
- FELSING STEFANIE ET AL: "A new approach in separating microplastics from environmental samples based on their electrostatic behavior", ENVIRONMENTAL POLLUTION, BARKING, GB, Bd. 234, 21. Dezember 2017 (2017-12-21), Seiten 20-28, XP085343803, ISSN: 0269-7491, DOI: 10.1016/J.ENVPOL.2017.11.013
- Köhnlechner R. ET AL: "Praktischer Einsatz elektrostatischer Separatoren in der Sekundärrohstoffindustrie", , 1. Januar 2009 (2009-01-01), Seiten 1-4, XP55845026, Gefunden im Internet: URL:https://link.springer.com/content/pdf/10.1007/s00501-009-0453-2.pdf [gefunden am 2021-09-27]
- Schubert G ET AL: "Beeinflussung der Kontaktaufladung von Kunststoffen durch Modifizierung ihrer Oberfläche", , 1. Januar 2004 (2004-01-01), Seiten 1-29, XP55845008, Gefunden im Internet: URL:http://www.ipfdd.de/fileadmin/user_upl oad/pg/arbeitsgebiete/elektrosortierung/Ab schlussberichtSFB285_2004.pdf [gefunden am 2021-09-27]
- SILVEIRA A V M ET AL: "Application of tribo-electrostatic separation in the recycling of plastic wastes", PROCESS SAFETY AND ENVIRONMENTAL PROTECTION, INSTITUTION OF CHEMICAL ENGINEERS, RUGBY, GB, Bd. 114, 3. Januar 2018 (2018-01-03), Seiten 219-228, XP085351506, ISSN: 0957-5820, DOI: 10.1016/J.PSEP.2017.12.019
- Weise Mandy ET AL: "Sächsischer Umweltpreis", , 4 July 2019 (2019-07-04), pages 1-3, XP55844053, Retrieved from the Internet: URL:https://www.researchgate.net/profile/K athrin-Harre/publication/336268564_Sachsis cher_Umweltpreis/links/5d97e46c92851c2f70e b9532/Saechsischer-Umweltpreis.pdf [retrieved on 2021-09-23]
- Schubert G ET AL: "Beeinflussung der Kontaktaufladung von Kunststoffen durch Modifizierung ihrer Oberfläche", , 1 January 2004 (2004-01-01), pages 1-29, XP55845008, Retrieved from the Internet: URL:http://www.ipfdd.de/fileadmin/user_upl oad/pg/arbeitsgebiete/elektrosortierung/Ab schlussberichtSFB285_2004.pdf [retrieved on 2021-09-27]
- Köhnlechner R. ET AL: "Praktischer Einsatz elektrostatischer Separatoren in der Sekundärrohstoffindustrie", , 1 January 2009 (2009-01-01), pages 1-4, XP55845026, Retrieved from the Internet: URL:https://link.springer.com/content/pdf/10.1007/s00501-009-0453-2.pdf [retrieved on 2021-09-27]

**Beschreibung**

Die Erfindung betrifft ein Verfahren zur Quantifizierung von Polymerspezies in einer Polymerpartikel enthaltenden Probe, insbesondere einer Sedimentprobe.

Dem allgegenwärtigen Einsatz von Polymeren folgt ein erheblicher Umwelteintrag in Form von Mikroplastik oder Mikrokunststoff, worunter Partikel verschiedener Polymerspezies mit einem Durchmesser kleiner als 5 mm zu verstehen sind. Während primäre Mikroplastikpartikel in reiner Form beispielsweise in kosmetischen Produkten oder in Reinigungsmitteln eingesetzt werden, können sekundäre Mikroplastikpartikel durch Faserabrieb bei der Textilwäsche, durch Abrieb von Autoreifen oder aufgrund von Korrosion größerer Polymerpartikel gebildet werden. Über verschiedene Verbreitungswege gelangen Mikroplastikpartikel schließlich in die Fließgewässer und in die Ozeane. Große Ansammlungen von Mikroplastikpartikeln sind daher in Flussauen und den Sedimenten von Ozeanen zu finden. Die ubiquitäre Verbreitung von Polymerpartikeln und die lange Zerfallsdauer von hunderten Jahren sowie die Fähigkeit Giftstoffe anzureichern, ist Gegenstand zahlreicher Forschungen und Untersuchungen. In diesem Zusammenhang werden reproduzierbare Analysemethoden zur Quantifizierung von Polymerspezies in einer Polymerpartikel enthaltenden Sedimentprobe gefordert. Bislang erfolgt die Analyse von Polymerpartikeln aus Sedimenten beginnend mit der Abtrennung von Kies und Sand sowie Schwebstoffen durch Dichtefraktionierung unter Zuhilfenahme von Arbeitslösungen, wie beispielsweise unterschiedlich dichten Salzlösungen. Organische Bestandteile werden chemisch oder enzymatisch zersetzt. Die aufbereitete Probe wird anschließend mikroskopisch oder thermodynamisch, beispielsweise mittels Differenzkalorimetrie (DCS), analysiert. Die Probenaufbereitung zur Quantifizierung von Polymerpartikeln nach bekannten Analysemethoden hat zum Nachteil, dass bereits im ersten Schritt größere Polymerpartikel abgetrennt und im zweiten Schritt die Form und Zusammensetzung der Polymerpartikel potentiell geändert wird, wodurch die nachfolgende Analyse verfälscht werden kann.

Für eine Massenbilanzierung werden bislang thermische Analysemethoden, wie Pyrolyse-Verfahren oder TED-GC/MS, verwendet. Die Abkürzung TED-GC-MS steht für ein zweistufiges Analyseverfahren, bei welchem eine Polymerpartikel enthaltende Probe in einem ersten Schritt vollständig pyrolisiert wird. Dabei werden die polymerspezifischen Zersetzungsprodukte auf eine Festphase gesammelt und in einem zweiten Schritt thermisch desorbiert, chromatographisch getrennt und abschließend mittels Massenspektrometrie bestimmt.

Die bekannten Quantifizierungsmethoden sind vergleichsweise zeitaufwändig sowie kostenaufwändig und weisen Fehlerpotentiale auf. Nachteilig ist weiterhin, dass die Wiederfindungsrate bei der Dichtefraktionierung mit zunehmend größeren Partikeln sinkt. Durch chemische und enzymatische Reinigungsmethoden können Masseverluste und Formverluste der Polymerpartikel auftreten, wodurch eine nachfolgende analytische Auswertung beeinträchtigt wird. Die Intensität der Verluste wird durch die verwendeten Chemikalien oder Enzyme und durch die Polymerspezies beeinflusst. So können insbesondere die Polymerspezies Polyamid und Celluloseacetat durch chemische und enzymatische Reinigungsschritte vollständig aufgelöst werden, wodurch die Massenbilanz einer Probe verfälscht wird.

Alternative Trennungsverfahren zum Separieren von Partikelgemischen beruhen auf dem Prinzip der elektrostatischen Trennung infolge einer Koronaaufladung beziehungsweise Koronaentladung oder infolge einer elektrischen Aufladung durch triboelektrische Effekte. Dabei werden Polymerpartikel zunächst elektrisch aufgeladen und anschließend in einem elektrischen Feld entsprechend ihrer Ladung separiert.

Aus der WO 2012 003 935 A1 ist ein Verfahren zur Elektrosortierung mittels Koronaentladung bekannt, wobei mehrere Fraktionen aufgrund unterschiedlicher elektrischer Leitfähigkeit erhalten werden.

Aus der DE 19901743 A1 sind ein Verfahren und eine Vorrichtung zur Trennung von Kunststoffen durch triboelektrische Aufladung und anschließende elektrostatische Trennung bekannt.

Gemäß DE 19901743 A1 werden Kunststoffe zunächst konditioniert und dabei in Teilchenform überführt. Die konditionierten Partikel werden einer Stoßbeanspruchung, Schlagbeanspruchung und Reibungsbeanspruchung zwischen zwei gleichmäßig beabstandeten Prallplatten ausgesetzt und dabei triboelektrisch positiv oder negativ aufgeladen. Die unterschiedlich triboelektrisch aufgeladenen Partikel werden anschließend einem positiv oder negativ gepolten elektrostatischen Feld ausgesetzt und dabei separiert.

Als nachteilig erweisen sich die bekannten Verfahren zur Elektroseparation von Partikeln aufgrund ihrer geringen Selektivität zur Quantifizierung von Partikelgemischen. Es wird daher ein Verfahren gefordert, welches eine selektive Quantifizierung von Polymerspezies in einer Polymerpartikel enthaltenden Probe ermöglicht.

Laura Cutroneo et al: "Microplastics in seawater: sampling strategies, laboratory methodologies, and identification techniques applied to port environment", Environmental Science and Polution Research, Bd. 27, Nr. 9, 6. Februar 2020, Seiten 8938-8952, ISSN: 0944-1344, DOI: 10.1007/S11356-020-07783-8 und Bin Zhang et al: "Research Progress of Microplastics in Freshwater Sediments in China", Environmental Science and Polution Research, Bd. 27, Nr. 25, 8. Juni 2020, Seiten 31046-31060, ISSN: 0944-1344, DOI: 10.1007/ S11356-020-09473-X offenbaren weitere bekannten Verfahren zur Quantifizierung von Polymerspezies in Polymerpartikel enthaltenden Proben, insbesondere in Sedimentproben.

Die Aufgabe der Erfindung besteht darin, ein Verfahren vorzuschlagen, mit welchem eine Quantifizierung von Polymerspezies in einer Polymerpartikel enthaltenden Probe mit geringer Fehlerquote möglich ist und mit welchem die aus dem Stand der Technik bekannten Nachteile überwunden werden können.

Die Aufgabe wird durch ein Verfahren mit den Merkmalen gemäß Patentanspruch 1 gelöst. Ausführungsvarianten sind in den abhängigen Patentansprüchen angegeben.

Dem erfindungsgemäßen Verfahren liegt die Erkenntnis zugrunde, dass bei der Elektroseparation von Polymerpartikeln mittels elektrischer Aufladung, beispielsweise durch triboelektrische Aufladung oder durch Koronaaufladung mittels einer Koronaelektrode, und anschließender elektrostatischer Trennung, beispielsweise in einem elektrostatischen Freifallabscheider, eine breite räumliche Streuung der Polymerpartikel hervorgerufen wird. Aufgrund der räumlichen Streuung werden Polymerpartikel bei der elektrostatischen Trennung, bei welcher üblicherweise beabstandet angeordnete Auffangbehälter eingesetzt werden, in mehreren Auffangbehältern verteilt angereichert, so dass mehrere Fraktionen einer oder mehrerer Polymerspezies erhalten werden. Somit können die Partikel einer Partikelspezies infolge der Elektroseparation mit unterschiedlichen Anteilen in den beabstandet angeordneten Auffangbehältern enthalten sein. Bei der Elektroseparation einer Probe mit Polymerpartikeln unterschiedlicher Polymerspezies werden somit mehrere Fraktionen erhalten, in welchen Polymerpartikel der unterschiedlichen Polymerspezies mit unterschiedlichen Anteilen angereichert sind. Es hat sich gezeigt, dass die infolge der elektrostatischen Trennung hervorgerufene räumliche Partikelstreuung, welche auch als Partikelverteilung bezeichnet werden kann, durch die bei einer Elektroseparation gewählten Separationsparameter, wie beispielsweise elektrische Aufladung, Partikelgeschwindigkeit und elektrische Spannung, beeinflusst wird. Als weitere die Elektroseparation beeinflussende Parameter können eine Partikelfeuchte, eine Luftgeschwindigkeit in einer Wirbelschicht, eine Partikelgröße und eine Luftgeschwindigkeit im Trennbereich als Separationsparameter berücksichtigt werden.

Es hat sich ferner gezeigt, dass die bei einer Elektroseparation hervorgerufene Partikelverteilung bei gleichbleibenden Separationsparametern wiederholbar konstant ist. Aufgrund der konstanten Partikelverteilung kann eine elektroseparierte Polymerspezies über mehrere Auffangbehälter verteilt beziehungsweise in mehreren Auffangbehältern unterschiedlich stark angereichert sein. Entsprechend der Anzahl der bei einer Elektroseparation eingesetzten Auffangbehälter können somit mehrere Fraktionen einer Polymerspezies erhalten werden. Durch die Anzahl der Auffangbehälter wird demnach die Anzahl möglicher Fraktionen festgelegt. Dementsprechend ist die Anzahl möglicher Fraktionen durch die Anzahl der Auffangbehälter vorgegeben. Ausgehend von einer Polymerpartikel enthaltenden Probe können somit nach der elektrostatischen Trennung in den vorgegebenen Auffangbehältern unterschiedliche Anteile von verschiedenen Polymerspezies enthalten sein, was eine Quantifizierung des Massenanteils von in einer Probe enthaltenden Polymerspezies erschwert.

Bei dem erfindungsgemäßen Verfahren wird daher die infolge einer elektrostatischen Trennung hervorgerufene räumliche Streuung oder Partikelverteilung verursachte Anreicherung einer Polymerspezies in mehreren vorgegebenen Fraktionen zugrunde gelegt, um jeweils nach elektrischer Aufladung und elektrostatischer Trennung zunächst einen polymerspezifischen Anreicherungsfaktor von reinen Polymerspezies für jede erhaltene Fraktionen, das heißt für jeden Auffangbehälter, zu bestimmen. Für eine vorgegebene Anzahl Auffangbehälter können aufgrund der konstanten Partikelverteilung somit den Auffangbehältern zuordenbare polymerspezifische Anreicherungsfaktoren bestimmt werden. Der polymerspezifische Anreicherungsfaktor eines Auffangbehälters entspricht dabei dem in dem Auffangbehälter nach elektrischer Aufladung und elektrostatischer Trennung enthaltenen Anteil einer zur Elektroseparation eingesetzten Menge einer Polymerspezies. Die Summe aus den polymerspezifischen Anreicherungsfaktoren ist demnach 1.

Um vorgegebene Fraktionen durch Elektroseparation einer Polymerspezies zu erhalten, werden vorgegebene Auffangbehälter eingesetzt, welche in vorgegebenem Abstand voneinander angeordnet werden, um elektrostatisch getrennte Polymerpartikel entsprechend ihrer Verteilung bei vorgegebenen Separationsparametern aufzufangen. Die Anzahl der Fraktionen wird demzufolge durch die Anzahl der Auffangbehälter bestimmt. Vorzugsweise werden mindestens drei Auffangbehälter eingesetzt, um mindestens drei Fraktionen zu erhalten.

Die Bereitstellung des polymerspezifischen Anreicherungsfaktors für unterschiedliche Polymerspezies erfolgt experimentell oder von einer Datenbank, wobei es sich bei den von einer Datenbank bereitgestellten polymerspezifischen Anreicherungsfaktoren ebenfalls um experimentell erworbene Daten handelt, welche für einen späteren Gebrauch gespeichert sind. Zur experimentellen Bestimmung des polymerspezifischen Anreicherungsfaktors einer Polymerspezies wird in einem ersten Schritt a) des erfindungsgemäßen Verfahrens zunächst eine Elektroseparation jeweils für verschiedene reine Polymerspezies eines vorgegebenen Partikelgrößenbereichs oder einer vorgegebenen Partikelgröße bei vorgegebenen Separationsparametern durchgeführt. Dabei können mindestens drei Auffangbehälter, vorzugsweise mindestens fünf Auffangbehälter eingesetzt werden. Die Bestimmung der polymerspezifischen Anreicherungsfaktoren von Polymerspezies kann für verschiedene Partikelgrößen oder verschiedene Partikelgrößenbereiche erfolgen. In Schritt a) wird also so vorgegangen, dass für verschiedene reine Polymerspezies eine Elektroseparation durchgeführt wird, wobei die Polymerspeziespartikel einer vorgegebenen Partikelgröße oder eines vorgegebenen Partikelgrößenbereichs bei vorgegeben Separationsparametern elektrisch aufgeladen und anschließend elektrostatisch in nebeneinander angeordneten Auffangbehältern getrennt werden, wobei für jeden Auffangbehälter ein polymerspezifischer Anreicherungsfaktor des in dem jeweiligen Auffangbehälter enthaltenen Anteils der getrennten Polymerspezies bestimmt wird.

Somit wird ein polymerspezifischer Anreicherungsfaktor einer Polymerspezies für jeden Auffangbehälter, das heißt für jede Fraktion, erhalten. Die in den jeweiligen erhaltenen Fraktionen enthaltenen Anteile der eingesetzten Partikelmenge oder der eingesetzten Partikelmasse können durch Zählen der in den Fraktionen enthaltenen Partikel oder durch Wägen der einzelnen Fraktionen erhalten werden.

Da die Bestimmung der polymerspezifischen Anreicherungsfaktoren bei vorgegeben Separationsparametern erfolgt, sind die bestimmten polymerspezifischen Anreicherungsfaktoren jeweils auch separationsparameterabhängig, so dass die polymerspezifischen Anreicherungsfaktoren auch als separationsparameterabhängige, polymerspezifische Anreicherungsfaktoren bezeichnet werden können. Der Einfachheit halber wird jedoch vornehmlich die Wendung polymerspezifische Anreicherungsfaktoren verwendet.

Mit der Bestimmung von polymerspezifischen Anreicherungsfaktoren für reine Polymerspezies bei konstanten Separationsparametern erfolgt in Schritt a) eine Referenzbestimmung zur Quantifizierung der in einer Polymerpartikel enthaltenden Probe enthaltenen Polymerspezies. Die polymerspezifischen Anreicherungsfaktoren werden somit anhand von vorgegebenen Proben ermittelt, deren Polymerpartikelmenge oder Polymerpartikelmasse bekannt ist. Soweit es sich um bekannte Polymerspezies handelt, stehen diese für die weitere Auswertung zur Quantifizierung zur Verfügung. Alternativ besteht gemäß Schritt b) des erfindungsgemäßen Verfahrens die Möglichkeit, dass die polymerspezifischen, separationsparameterabhängigen Anreicherungsfaktoren von verschiedenen reinen Polymerspezies von einer Datenbank bereitgestellt werden. In einer solchen Datenbank können polymerspezifische Anreicherungsfaktoren, welche nach vorgegebenen Separationsparametern experimentell ermittelt worden sind, enthalten sein. Experimentell ermittelte polymerspezifische Anreicherungsfaktoren können in der Datenbank gespeichert werden.

In einem Schritt c) des erfindungsgemäßen Verfahrens wird eine Polymerpartikel enthaltende Probe, bestehend aus Partikeln einer vorgegebenen Partikelgröße oder eines vorgegebenen Partikelgrößenbereichs, bereitgestellt. Die Bereitstellung kann eine Vorseparierung zur Abtrennung von Sediment und Schwebteilchen umfassen. Die Vorseparierung kann mit Hilfe einer Koronaelektrode erfolgen. Dabei wird eine Umweltprobe, beispielsweise eine Sedimentprobe, dem Einflussbereich der Koronaelektrode ausgesetzt, wobei in der Umweltprobe enthaltene aufladbare Partikel, wie Polymerpartikel, elektrisch aufgeladen werden. Anschließend werden die elektrisch aufgeladenen Partikel von den nicht aufladbaren Partikeln in einem elektrischen Feld getrennt. Im Ergebnis der Vorseparierung wird eine mit Polymerpartikeln angereicherte Probe erhalten. Auf diese Weise können bei der Probenbereitstellung der Polymerpartikel enthaltenden Probe nicht-aufladbare Bestandteile abgetrennt werden. Da die Restfeuchte der Polymerpartikel enthaltenden Probe einen Einfluss auf die Trennleistung bei der elektrostatischen Trennung hat, sollte die Restfeuchte für jede Elektroseparation gleich sein. Eine vorgegebene Restfeuchte kann als Separationsparameter berücksichtigt werden. Zweckmäßigerweise kann zur Probenbereitstellung eine Trocknung der Polymerpartikel enthaltenden Probe vorgesehen werden.

In einem dritten Schritt d) des erfindungsgemäßen Verfahrens wird die bereitgestellte Polymerpartikel enthaltende Probe bei den vorgegebenen Separationsparametern elektrisch aufgeladen und elektrostatisch getrennt, wobei sich Anteile der Polymerpartikel enthaltenden Probe in den beabstandet nebeneinander angeordneten Auffangbehältern anreichern. Die Separationsparameter, nach welchen die Polymerpartikel enthaltende Probe getrennt beziehungsweise fraktioniert wird, entsprechen dabei den Separationsparametern von Schritt a). Da auch die Partikelgröße einen Einfluss auf die Trennleistung bei der elektrostatischen Trennung hat, sollten die Partikelgrößen oder die Partikelgrößenbereiche bei der Elektroseparation der reinen Polymerspezies zur Bestimmung der polymerspezifischen Anreicherungsfaktoren in Schritt a) und bei der Elektroseparation der Polymerpartikel enthaltenden Probe in Schritt d) im Wesentlichen übereinstimmen.

In einem vierten Schritt e) des erfindungsgemäßen Verfahrens wird für jeden Auffangbehälter eine spezifische Enthalpie und/oder eine spezifische Wärmekapazitätsänderung der in dem jeweiligen Auffangbehälter angereicherten Polymerpartikel der Polymerpartikel enthaltenden Probe bestimmt.

In einem fünften Schritt f) werden aus den jeweils bestimmten spezifischen Enthalpien und/oder den bestimmten spezifischen Wärmekapazitätsänderungen und den polymerspezifischen Anreicherungsfaktoren die Massenanteile der in der Polymerpartikel enthaltenden Probe enthaltenen Polymerspezies bestimmt. Die Quantifizierung einer Polymerspezies in einer Probe erfolgt über die Bestimmung der Wärmekapazitätsänderung $\Delta C_{p,exp}$ bei amorphen sowie der Schmelz- und Kristallisationsenthalpie $\Delta H_{exp}$ bei teilkristallinen Polymeren im Gemisch mit Kenntnis der spezifischen $\Delta c_{p,P}$- Wärmekapazitätsänderung und $\Delta h_P$- Schmelz- und Kristallisationsenthalpie der reinen Polymerspezies.

Bedingung für präzise Messergebnisse sind neben einer stets aktuellen Kalibrierung insbesondere identische Separationsparameter. Für jede polymerspezifische Auswertung gilt

$$m_P = \frac{\Delta H_{exp}}{\Delta h_P} = \frac{\Delta C_{p,exp}}{\Delta c_{p,P}}$$

für die Berechnung der Masse der Polymerspezies in einer Probe.

Der Massenanteil des Polymers in der Probe berechnet sich mit

$$w_P = \frac{m_P}{\sum m_i} \text{ und demzufolge mit } w_P = \frac{\Delta H_{exp}}{\sum m_i \, \Delta h_P}.$$

Die elektrische Aufladung zur Elektroseparation von Polymerpartikeln kann durch Koronaaufladung mittels einer Koronaelektrode oder triboelektrisch mit einer strömungsmechanischen Aufladeeinheit oder an einer rotierenden Metalltrommel erfolgen. Die elektrostatische Trennung der elektrisch aufgeladenen Partikel kann in einem elektrischen Feld, beispielsweise in einem elektrostatischen Freifallabscheider erfolgen. Dabei hat die elektrische Spannung zur Erzeugung des elektrischen Feldes einen Einfluss auf die Trennungsleistung zur Separation der Partikel in die vorgegebenen Auffangbehälter. Die bei der elektrostatischen Trennung verwendete elektrische Spannung ist daher ein Separationsparameter und wird entsprechend vorgegeben. Die Spannungen zur elektrostatischen Trennung sind in den Schritten a) und d) identisch.

Weiterhin kann die Trennung von Polymerpartikeln im Fall einer Koronaufladung an einer rotierenden Metalltrommel durch die Rotationsgeschwindigkeit der Metalltrommel beeinflusst werden. Die Rotationsgeschwindigkeit der Metalltrommel kann daher als Separationsparameter vorgegeben werden. Insofern ist die Rotationsgeschwindigkeit der Metalltrommel in den Schritten a) und d) identisch.

Bei der triboelektrischen Aufladung werden die Polymerpartikel einer Stoßbeanspruchung, Schlagbeanspruchung und/oder Reibungsbeanspruchung ausgesetzt und triboelektrisch positiv oder negativ aufgeladen. Die unterschiedlich triboelektrisch aufgeladenen Partikel werden anschließend einem positiv oder negativ gepolten elektrostatischen Feld ausgesetzt und dabei in die in nebeneinander angeordneten Auffangbehälter getrennt und somit in verschiedene Fraktionen separiert.

Die Partikelgröße der Polymerpartikel enthaltenden Probe kann im Rahmen der Probenbereitstellung durch mechanische Zerkleinerung, vorzugsweise durch Mahlen, besonders bevorzugt durch Kryomahlen, angepasst werden. Für die Elektroseparation sollte die mittlere Partikelgröße der Polymerpartikel enthaltenden Probe kleiner als 5 mm sein. Es kann daher vorgesehen werden, dass die Polymerpartikel enthaltende Probe mechanisch zerkleinert wird, bis eine mittlere Partikelgröße kleiner als 5 mm erreicht ist. Für die Durchführung der Elektroseparation kann eine Polymerpartikel enthaltende Probe derart vorbehandelt werden, dass die Partikel der Probe im Wesentlichen in einem Bereich kleiner 5 mm bis 1 mm liegen. Ferner kann eine Vorbehandlung der Polymerpartikel enthaltenden Probe vorgesehen werden, um Partikel bereitzustellen, welche eine Größe von 1 mm nicht überschreiten. Es kann daher eine Vorbehandlung der Polymerpartikel enthaltenden Probe vorgesehen werden, bei welcher Partikel bereitgestellt werden, welche eine Größe von 1 mm nicht überschreiten. Für den Fall, dass eine Vorbehandlung einer Probe hinsichtlich einer Partikelgröße oder eines Partikelgrößenbereichs nicht erwünscht ist, kann vorgesehen werden, dass eine mittlere Partikelgröße der Probe bestimmt wird, wobei die bestimmte mittlere Partikelgröße der Probe als ein Separationsparameter berücksichtigt wird. Gemäß einer Ausführungsvariante des Verfahrens kann daher vorgesehen werden, dass vor der Elektroseparation der Polymerpartikel enthaltenden Probe eine mittlere Partikelgröße der Polymerpartikel enthaltenden Probe bestimmt wird, wobei die bestimmte mittlere Partikelgröße bei der Bestimmung der polymerspezifischen Anreicherungsfaktoren gemäß Verfahrensschritt a) berücksichtigt wird. Mit anderen Worten kann die Partikelgröße bei der Bestimmung der polymerspezifischen Anreicherungsfaktoren gemäß Verfahrensschritt a) an die bestimmte mittlere Partikelgröße der Polymerpartikel enthaltenden Probe angepasst werden. Demzufolge sollte eine Bestimmung der mittleren Partikelgröße einer Polymerpartikel enthaltenden Probe im Rahmen der Probenbereitstellung vor dem Verfahrensschritt a) bestimmt werden, wenn unter bestimmten Gegebenheiten keine mechanische Probenvorbehandlung, beispielsweise durch Mahlen, gewünscht ist. Eine Bestimmung der mittleren Partikelgröße einer Polymerpartikel enthaltenden Probe im Rahmen der Probenbereitstellung vor dem Verfahrensschritt a) ist dabei nur dann erforderlich, wenn keine polymerspezifischen Anreicherungsfaktoren für verschiedene Polymerspezies bezüglich der bestimmten mittleren Partikelgröße vorliegen.

Die Bestimmung des polymerspezifischen Anreicherungsfaktors kann für verschiedene Polymerspezies für verschiedene Partikelgrößenbereiche vorgesehen werden. So kann eine Bestimmung des polymerspezifischen Anreicherungsfaktors für Polymerspeziesproben reiner Polymerspezies durchgeführt werden, welche aus Partikeln mit einer Größe im Bereich von 5 mm bis 1 mm, 1 mm bis 0,1 mm, 0,1 mm bis 0,01 mm, 0,01 mm bis 0,001 mm bestehen, wobei eine zu quantifizierende Polymerpartikel enthaltende Probe derart vorbehandelt wird, dass die zu quantifizierende Polymerpartikel enthaltende Probe aus Partikeln mit einer Größe im Bereich von 5 mm bis 1 mm, 1 mm bis 0,1 mm, 0,1 mm bis 0,01 mm oder 0,01 mm bis 0,001 mm besteht.

Für die Elektroseparation hat sich weiterhin ein Partikelgrößenbereich mit Partikeln im Bereich von 200 μm bis 63 μm als vorteilhaft erwiesen. Es kann daher vorgesehen werden, dass der Verfahrensschritt a) zur Bestimmung des polymerspezifischen Anreicherungsfaktors für verschiedene reine Polymerspeziesproben durchgeführt wird, welche aus Partikeln mit einer Größe im Bereich von 200 μm bis 63 μm besteht. Im Rahmen der Probenbereitstellung sollte demnach die Polymerpartikel enthaltende Probe derart vorbehandelt werden, dass Partikel mit einer Größe im Bereich von 200 μm bis 63 μm erhalten werden.

Zum Erreichen des vorgegebenen Partikelgrößenbereichs oder der vorgegebenen Partikelgröße kann vorgesehen werden, dass die Polymerpartikel enthaltende Probe und/oder reine Polymerspeziespartikel gemahlen, vorzugsweise kryogemahlen, wird/werden. Das Mahlen der Polymerpartikel enthaltenden Probe kann im Rahmen der Probenbereitstellung als Probenvorbehandlung durchgeführt werden. Das Mahlen von reinen Polymerspeziespartikeln kann vorgesehen werden, um Partikel einer vorgegebenen Größe oder eines vorgegebenen Größenbereichs, beispielsweise im Bereich von 5 mm bis 1 mm, 1 mm bis 0,1 mm, 0,1 mm bis 0,01 mm oder 0,01 mm bis 0,001 mm zu erhalten, um für verschiedene Partikelgrößen oder Partikelgrößenbereiche einer Polymerspezies eine Elektroseparation zur Bestimmung der polymerspezifischen Anreicherungsfaktoren durchführen zu können.

Als Separationsparameter können eine Feuchte, eine Polymerpartikelgröße, eine elektrische Spannung, eine elektrische Feldstärke, eine Vibrationsstärke und/oder eine Geschwindigkeit berücksichtigt werden.

Gemäß einer Ausführungsvariante des erfindungsgemäßen Verfahrens kann vorgesehen werden, dass eine Art und eine Anzahl der in der Polymerpartikel enthaltenden Probe enthaltenen verschiedenen Polymerspezies bestimmt wird. Eine Bestimmung derart und der Anzahl der in der Polymerpartikel enthaltenden Probe enthaltenen verschiedenen Polymerspezies kann mittels Differenzkalorimetrie, Pyrolyse-GC-MS, FTIR-Spektroskopie, IR-Spektroskopie, Raman-Mikroskopie oder TED-GC-MS erfolgen.

Bei der Bestimmung der spezifischen Enthalpien und/oder den bestimmten spezifischen Wärmekapazitätsänderungen der in Auffangbehältern separierten Anteile einer Polymerpartikel enthaltenden Probe kann es bei strukturell ähnlichen Polymerspezies zu einer Überlagerung der thermischen Phasenübergänge kommen, was eine Unterscheidung zwischen den strukturell ähnlichen Polymerspezies erschwert und bei der Quantifizierung ein verfälschtes Ergebnis zur Folge haben kann. Für den Fall, dass bei der Bestimmung der spezifischen Enthalpien und/oder den bestimmten spezifischen Wärmekapazitätsänderungen eine Überlagerung von thermischen Phasenübergängen festgestellt wird, kann vorgesehen werden, dass die Elektroseparation der Polymerpartikel enthaltenden Probe mit geänderten Separationsparametern und/oder einer größeren Anzahl von Auffangbehältern durchgeführt wird. Indem die Separationsparameter und/oder die Anzahl der Auffangbehälter verändert wird, kann die Anzahl der Fraktionen beeinflusst werden, so dass eine größere Auflösung erzielt werden kann. Ferner kann vorgesehen werden, dass eine zusätzliche Fraktionierung der in den betreffenden Auffangbehältern angereichten Polymerspezies bei geänderten Separationsparametern durchgeführt werden, wobei zur Quantifizierung der zu unterscheidenden Polymerspezies entsprechend der geänderten Separationsparameter angepasste polymerspezifische Anreicherungsfaktoren eingesetzt werden müssen.

Gemäß einer Ausführungsvariante des erfindungsgemäßen Verfahrens kann ferner vorgesehen werden, dass zumindest eine erhaltene Fraktion der Polymerpartikel enthaltenden Probe einer weiteren Separation mit geänderten Separationsparametern unterzogen wird. Indem eine erhaltene Fraktion der Polymerpartikel enthaltenden Probe einer weiteren Separation mit den geänderten Separationsparametern unterzogen wird, können solche Polymerspezies separiert werden, welche bei der Bestimmung der spezifischen Enthalpie und/oder der spezifischen Wärmekapazitätsänderung nicht eindeutig unterschieden werden können. Zweckmäßigerweise sollten polymerspezifische Anreicherungsfaktoren für strukturell ähnliche Polymerspezies oder für Polymerspezies, welche bei der Bestimmung der spezifischen Enthalpie oder der spezifischen Wärmekapazitätsänderung eine Signalüberlagerung verursachen, für unterschiedliche Separationsparameter bestimmt oder bereitgestellt werden.

Gemäß einer weiteren Ausführungsvariante des erfindungsgemäßen Verfahrens kann vorgesehen werden, dass die Separationsparameter, nach welchen die Polymerspeziespartikel getrennt werden, polymerspezifisch angepasst werden. Das heißt, dass die Separationsparameter jeweils an eine Polymerspezies angepasst werden.

Die spezifische Enthalpie und/oder eine spezifische Wärmekapazitätsänderung wird/werden bevorzugt mittels Differenzkalorimetrie bestimmt.

Gemäß einer noch weiteren Ausführungsvariante des erfindungsgemäßen Verfahrens kann vorgesehen werden, dass eine Polymerpartikel enthaltende Probe zur Probenbereitstellung in einem ersten Separationsschritt mittels Koronaaufladung in aufladbare und nicht-aufladbare Bestandteile getrennt wird, wobei die aufladbaren Bestandteile, wobei es sich um die polymerreiche Partikelfraktion handelt, in einem zweiten Separationsschritt triboelektrisch aufgeladen und anschließend in einem elektrischen Feld separiert werden. Dabei kann der erste Separationsschritt als Grobseparation und der zweite Separationsschritt als Feinseparation verstanden werden.

Weitere Einzelheiten, Merkmale und Vorteile von Ausführungsvarianten der Erfindung ergeben sich aus der nachfolgenden Beschreibung zweier Ausführungsbeispiele.

Das erfindungsgemäße Verfahren zur Quantifizierung von Polymerspezies in einer Polymerpartikel enthaltenden Probe umfasst mehrere Schritte. In einem Schritt a) werden zunächst polymerspezifische Anreicherungsfaktoren von verschiedenen reinen Polymerspezies mit vorgegebener Partikelgröße durch Elektroseparation bei vorgegebenen Separationsparametern bestimmt. Dabei werden jeweils eine vorgegebene Ausgangsmenge Partikel reiner Polymerspezies mit vorgegebener Partikelgröße bei den vorgegebenen Separationsparametern in drei Auffangbehälter separiert. Anschließend wird für jeden Auffangbehälter eine Anreicherung der separierten Polymerspezies bestimmt, wobei, bezogen auf die vorgegebene Ausgangsmenge, für jeden Auffangbehälter ein polymerspezifischer Anreicherungsfaktor bestimmt wird. Alternativ können die polymerspezifischen Anreicherungsfaktoren gemäß Schritt b) von einer Datenbank bereitgestellt werden, wobei die Separationsparameter vorgegeben sind. Schritt a) entfällt in diesem Fall.

In einem Schritt c) wird eine Polymerpartikel enthaltende Probe bereitgestellt. Dabei kann vorgesehen werden, dass die Polymerpartikel enthaltende Probe bis zum Erreichen einer vorgegeben Feuchte getrocknet wird. Ferner kann vorgesehen werden, dass die Polymerpartikel enthaltende Probe gemahlen wird, um eine vorgegebene mittlere Partikelgröße oder einen vorgegebenen Partikelgrößenbereich zu gewährleisten.

Anschließend wird die Polymerpartikel enthaltende Probe in einem Schritt d) mit den vorgegebenen Separationsparametern separiert und in einem Schritt e) für jeden Auffangbehälter eine spezifische Enthalpie und/oder eine spezifische Wärmekapazitätsänderung der darin angereicherten Polymerpartikel bestimmt. Zur Quantifizierung der in der Probe enthaltenen Polymerspezies werden in einem Schritt f) aus den bestimmten spezifischen Enthalpien und/oder der bestimmten spezifischen Wärmekapazitätsänderungen unter Berücksichtigung der in Schritt a) bestimmten polymerspezifischen Anreicherungsfaktoren die Massenanteile der in der Polymerpartikel enthaltenden Probe enthaltenen Polymerspezies bestimmt.

Im Einzelnen kann dabei wie folgt vorgegangen werden. Die Quantifizierung der Polymerspezies in der Polymerpartikel enthaltenden Probe erfolgt über die Bestimmung der spezifischen Wärmekapazitätsänderung $\Delta c_{p,exp}$ bei amorphen Polymerspezies sowie der spezifischen Schmelz- und Kristallisationsenthalpie $\Delta H_{exp}$ bei teilkristallinen Polymerspezies im Gemisch mit Kenntnis der $\Delta c_{p,P}$-Werte und der $\Delta h_P$-Werte der reinen Polymerspezies. Die Trennungsparameter zur Ermittlung der $\Delta c_{p,P}$-Werte und der $\Delta h_P$-Werte sind polymerspezifisch und können anhand der Vorversuche gemäß Schritt a) des erfindungsgemäßen Verfahrens bestimmt und festgelegt werden. Für präzise Messergebnisse sind stets identische Separationsparameter einzuhalten. Für jede polymerspezifische Quantifizierung gilt

$$w_P = \frac{\Delta h_{exp}}{\Delta h_P} = \frac{\Delta c_{p,exp}}{\Delta c_{p,P}}$$

für die Berechnung des Massenanteils einer Polymerspezies in der elektroseparierten Polymerpartikel enthaltenden Probe, wobei $w_P$ der Massenanteil der jeweiligen Polymerspezies in der untersuchten Polymerpartikel enthaltenden Probe ist.

Gemäß einem ersten Ausführungsbeispiel werden 10 mg einer Probe, welche eine unbekannte Menge eines Polyamides (PA) enthält, separiert. Dabei ist in der Heizkurve einer durchgeführten dynamischen Differenzkalorimetrie im Temperaturbereich von 200 bis 250 °C ein Schmelzübergang zu entnehmen. Die spezifische Schmelzenthalpie $\Delta h_P$ dieses Polymers $\Delta h_{P,PA}$ beträgt 58,3 J/g. Es wird in der zweiten Aufheizkurve eine Schmelzenthalpie von 175 mJ gemessen. Die Masse an PA berechnet sich nach

$$m_P = \frac{\Delta h_{exp}}{\Delta h_P} = \frac{175\,mJ}{58,3\,J/g} = 3\,mg.$$

Der Massenanteil des Polymers in der Probe berechnet sich zu $w_P = \frac{m_P}{\sum m_i}$ und damit

$$w_P = \frac{\Delta h_{exp}}{\sum m_i\,\Delta h_P} = \frac{175\,mJ}{10\,mg \cdot 58,3\,J/g} = 0{,}3$$ beziehungsweise 30%.

Bei der Elektroseparation einer Polymerpartikel enthaltenden Probe kann es vorkommen, dass in einem Auffangbehälter Anteile von unterschiedlichen Polymerspezies angereichert werden, welche bei der Differenzkalorimetrie eine Überlagerung der thermischen Phasenübergänge verursachen. Gemäß einer Ausführungsvariante der Elektroseparation ist eine Quantifizierung auch bei einer Überlagerung der thermischen Phasenübergänge möglich. Dies ist im Folgenden anhand eines Beispiels einer Probe mit zwei Polymerspezies unterschiedlicher chemischer Natur beschrieben, wobei die zwei Polymerspezies sich überlagernde Schmelzkurven zeigen. Für den Fall, dass die zu untersuchende Polymerpartikel enthaltende Probe zwei Polymerspezies aufweist, gilt

$$m_{P,ges} = m_{P1} + m_{P2}$$

mit der Masse der gesamten enthaltenen Polymermenge $m_{p,ges}$, mit der Masse der enthaltenen ersten Polymerspezies $m_{P1}$ und der Masse der enthaltenen zweiten Polymerspezies $m_{P2}$.

Bei zwei Polymerspezies gilt für die Massenanteile:

$$w_{P,ges} = w_{P1} + w_{P2} = 1$$

Bei der Differenzkalorimetrie wird bei überlagernden Phasenübergängen der beiden Polymerspezies nur ein Phasenübergangsignal gemessen, welches sich aus der spezifischen Enthalpie $\Delta h_P$ der jeweiligen Polymerspezies - erste oder zweite Polymerspezies - und dem Massenanteil $w_{Pi}$ der jeweiligen Polymerspezies ergibt

$$\Delta h_{P,ges} = w_{P1} \cdot \Delta h_{P1} + w_{P2} \cdot \Delta h_{P2}.$$

Durch Separation mittels triboelektrischer Aufladung werden aus der Gesamtprobe zunächst zwei Fraktionen mit unterschiedlichen Anteilen der jeweiligen Polymerspezies erhalten. Dies kann in einem Schritt innerhalb der Separation des Polymeranteils aus der zu untersuchenden Probe oder in einem weiteren Separationsschritt erfolgen. Besonders vorteilhaft ist dabei die Kombination aus einer Grobseparation mittels Koronaaufladung und einer nachfolgenden Feinseparation der polymerreichen Fraktion mittels triboelektrischer Aufladung. Aus der ursprünglich vorhandenen Masse wird der nun in der Fraktion vorhandene Anteil, das heißt der in dem Auffangbehälter vorhandene Anteil, durch einen Anreicherungsfaktor s beschrieben. Dieser ist durch die jeweiligen Separationsparameter, wie beispielsweise Feuchte, Partikelgröße, elektrische Spannung, elektrische Feldstärke, Vibrationsstärke der Vibrationsrinne, Rotationsgeschwindigkeit einer Metallwalze, für die Fraktion und die Eigenschaften der Polymerspezies bestimmt. Für jeweils festgelegte Separationsparameter sind diese Anreicherungsfaktoren nur noch von der chemischen Natur der Polymerspezies abhängig und demzufolge polymerspezifisch.

Bei zwei Polymerspezies und zwei Fraktionen ergeben sich vier Anreicherungsfaktoren $s_{P1F1}$ für die erste Polymerspezies in der ersten Fraktion, $s_{P1F2}$ für die erste Polymerspezies in der zweiten Fraktion, $s_{P2F1}$ für die zweite Polymerspezies in der ersten Fraktion und $s_{P2F2}$ für die zweite Polymerspezies in der zweiten Fraktion. Die erhaltenen Anreicherungsfaktoren, welche gemäß Schritt a) des erfindungsgemäßen Verfahrens bestimmt werden, können mit Hilfe von Aufstockungsproben oder Spikeproben bekannten Polymergehaltes unabhängig von der zu quantifizierenden Probe bestimmt werden. Geeignete Separationsparameter können experimentell bestimmt werden. Mit Hilfe der Separation werden aus der zu untersuchenden Probe mindestens zwei Fraktionen mit unterschiedlichen Anteilen der beiden Polymerspezies erhalten. Zur Messung in der DSC stehen nun zwei Proben zur Verfügung, deren Masse $m_{F1}$ und $m_{F2}$ durch Wägung bestimmt werden können und über die jeweiligen Anreicherungsfaktoren mit den beiden unbekannten Massen $m_1$ und $m_2$ in der Gesamtprobenmenge verknüpft sind:

Masse an Polymer 1 in Fraktion 1: $m_{11} = s_{11}m_1$  Gleichung 1

Masse an Polymer 1 in Fraktion 2: $m_{12} = s_{12}m_1$  Gleichung 2

Masse an Polymer 2 in Fraktion 1: $m_{21} = s_{21}m_2$  Gleichung 3

Masse an Polymer 2 in Fraktion 2: $m_{22} = s_{22}m_2$  Gleichung 4

Die Massen der jeweiligen Fraktionen sind über Wägung zu ermitteln und stehen über die Anreicherungsfaktoren in Zusammenhang mit der Masse der Polymere in der Ausgangsprobe.

$$m_{F1} = s_{11}m_1 + s_{21}m_2 \text{ und } m_{F2} = s_{12}m_1 + s_{22}m_2.$$  Gleichung 5

Für die Gesamtmasse gilt:

$$m_{P,ges} = m_{F1} + m_{F2}$$  Gleichung 6

Zur Masse des jeweiligen Polymers in der Ausgangsprobe bestehen folgende Beziehungen:

$$m_1 = s_{11}m_1 + s_{12}m_1$$  Gleichung 7

$$m_2 = s_{21}m_2 + s_{22}m_2 \hspace{3cm} \text{Gleichung 8}$$

In der Praxis können zusätzlich apparative Verluste der Gesamtmasse der Probe auftreten, die entsprechend in der Gesamtmassenbilanz zu berücksichtigen sind.

Mittels DSC wird nun für jede erhaltene Fraktion die Enthalpie $\Delta H_P$ der Fraktion gemessen. Diese setzt sich aus den Summen der Produkte der neuen Massenanteile des jeweiligen Polymers, welche über den Anreicherungsfaktor in Bezug zur ursprünglichen Masse beziehungsweise zum Massenanteil in der Ausgangsprobe stehen und dessen spezifischer Enthalpie, zusammen.

$$\text{Für Fraktion 1 gilt:} \quad \Delta H_{F1} = s_{11}m_1\Delta h_1 + s_{21}m_2\Delta h_2 \hspace{2cm} \text{Gleichung 9}$$

$$\text{Für Fraktion 2 gilt:} \quad \Delta H_{F2} = s_{12}m_1\Delta h_1 + s_{22}m_2 \ \ \Delta h_2 \hspace{2cm} \text{Gleichung 10}$$

Da sowohl die Anreicherungsfaktoren als auch die spezifischen Enthalpien der Polymere 1 und 2 bekannt sind, können diese als Konstante für die jeweilige Fraktion gewertet werden. Die Gesamtenthalpien der Fraktionen 1 und 2, $\Delta H_{F1}$ und $\Delta H_{F2}$, wurden gemessen und sind damit bekannt. Anschließend werden die zwei Gleichungen mit den zwei Unbekannten $m_1$ und $m_2$ nach dem Additions-, Einsetz- oder Gleichsetzungsverfahren berechnet.

Für Fraktion 1 gilt:

$$m_1 \quad = \frac{\Delta H_{F1} - s_{21}m_2\Delta h_2}{\Delta h_1 s_{11}} \hspace{3cm} \text{Gleichung 11}$$

Für Fraktion 2 gilt:

$$m_1 \quad = \frac{\Delta H_{F2} - s_{22}m_2\Delta h_2}{\Delta h_1 s_{12}} \hspace{3cm} \text{Gleichung 12}$$

Gleichsetzen führt zu:

$$\frac{\Delta H_{F2} - s_{22}m_2\Delta h_2}{\Delta h_1 s_{12}} = \frac{\Delta H_{F1} - s_{21}m_2\Delta h_2}{\Delta h_1 s_{11}} \hspace{2cm} \text{Gleichung 13}$$

Kürzen führt zu:

$$\frac{\Delta H_{F2} - s_{22}m_2\Delta h_2}{s_{12}} = \frac{\Delta H_{F1} - s_{21}m_2\Delta h_2}{s_{11}} \hspace{2cm} \text{Gleichung 14}$$

Erweitern mit $s_{12}s_{11}$

$$(\Delta H_{F2} - s_{22}m_2\Delta h_2)s_{11} = (\Delta H_{F1} - s_{21}m_2\Delta h_2)s_{12} \hspace{1.5cm} \text{Gleichung 15}$$

Anschließendes Ausmultiplizieren und Umstellen nach $m_2$ führt zu einer Bestimmungsgleichung für die Masse $m_2$.

$$s_{11}\Delta H_{F2} - s_{11}s_{22}m_2\Delta h_2 = s_{12}\Delta H_{F1} - s_{12}s_{21}m_2\Delta h_2 \hspace{1cm} \text{Gleichung 16}$$

$$s_{12}s_{21}m_2\Delta h_2 - s_{11}s_{22}m_2\Delta h_2 = s_{12}\Delta H_{F1} - s_{11}\Delta H_{F2} \hspace{1cm} \text{Gleichung 17}$$

$$m_2\left(s_{12}s_{21}\ \Delta h_2 - s_{11}s_{22}\ \Delta h_2\right) = s_{12}\Delta H_{F1} - s_{11}\Delta H_{F2} \qquad \text{Gleichung 18}$$

$$m_2 = \frac{s_{12}\Delta H_{F1}-s_{11}\Delta H_{F2}}{s_{12}s_{21}\ \Delta h_2-s_{11}s_{22}\ \Delta h_2} \qquad \text{Gleichung 19}$$

Ebenso kann daraus $m_1$ wie folgt bestimmt werden:

$$\text{Für Fraktion 1 gilt:} m_2 = \frac{\Delta H_{F1}-\ s_{11}m_1\Delta h_1}{s_{21}\ \Delta h_2} \qquad \text{Gleichung 20}$$

$$\text{Für Fraktion 2 gilt:} m_2 = \frac{\Delta H_{F2}-\ s_{12}m_1\Delta h_1}{s_{22}\ \Delta h_2} \qquad \text{Gleichung 21}$$

Gleichsetzen:

$$\frac{\Delta H_{F2}-\ s_{12}m_1\Delta h_1}{s_{22}} = \frac{\Delta H_{F1}-\ s_{11}m_1\Delta h_1}{s_{21}}, \qquad \text{Gleichung 22}$$

Erweitern und Kürzen

$$s_{22}\ \ (\Delta H_{F1}-\ s_{11}m_1\Delta h_1) = s_{21}\ \ (\Delta H_{F2}-\ s_{12}m_1\Delta h_1) \qquad \text{Gleichung 23}$$

Ausmultiplizieren:

$$s_{22}\ \ \Delta H_{F1} - s_{22}s_{11}m_1\Delta h_1 = s_{21}\ \ \Delta H_{F2} - s_{21}s_{12}m_1\Delta h_1 \qquad \text{Gleichung 24}$$

$$s_{22}\ \ \Delta H_{F1} - s_{21}\ \ \Delta H_{F2} = m_1\left(s_{22}s_{11}\ \ \Delta h_1 - s_{21}s_{12}\ \ \Delta h_1\right) \qquad \text{Gleichung 25}$$

$$m_1 = \frac{s_{22}\ \ \Delta H_{F1}-s_{21}\ \ \Delta H_{F2}}{s_{22}s_{11}\ \ \Delta h_1-s_{21}s_{12}\ \ \Delta h_1} \qquad \text{Gleichung 26}$$

Diese hat die gleiche Form wie:

$$m_2 = \frac{s_{12}\Delta H_{F1}-s_{11}\Delta H_{F2}}{s_{12}s_{21}\ \Delta h_2-s_{11}s_{22}\ \Delta h_2} \qquad \text{Gleichung 19}$$

Die Faktoren der Schmelz- beziehungsweise Kristallisationsenthalpien ($\Delta h_i, \Delta H_{Fi}$) in Gleichung 19 und 26 lassen sich zudem durch die Wärmekapazitätsänderung ($\Delta c_i, \Delta C_{Fi}$) substituieren.

$$m_1 = \frac{s_{22}\ \ \Delta C_{F1}-s_{21}\ \ \Delta C_{F2}}{s_{22}s_{11}\ \ \Delta c_1-s_{21}s_{12}\ \ \Delta c_1} \qquad \text{Gleichung 27}$$

$$m_2 = \frac{s_{12}\ \ \Delta C_{F1}-s_{11}\ \ \Delta C_{F2}}{s_{12}s_{21}\ \ \Delta c_2-s_{11}s_{22}\ \ \Delta c_2} \qquad \text{Gleichung 28}$$

Die vorhergehenden Ausführungen sind mit nachfolgendem Beispiel näher erläutert. Eine Probe zeigt einen Kristallisationspeak im Temperaturbereich von 125 bis 90 °C. Phasenübergänge in diesem Bereich können sowohl dem Polymer 1, wobei es sich um Polyethylen (PE) handelt, als auch dem Polymer 2, wobei es sich um Polypropylen (PP) handelt, zugeordnet werden. Bei der Elektroseparation der Probe werden zwei Fraktionen erhalten. Die Anreicherungsfaktoren für die jeweiligen Polymere 1 und 2 für die gewählten Separationsparameter werden gesondert bestimmt. Die Anreicherungsfaktoren werden für Polymer 1 mit $s_{11}=0,4$ in Fraktion 1 und $s_{12}=0,6$ in Fraktion 2 bestimmt. Für Polymer 2 werden die Anreicherungsfaktoren mit $s_{21}=0,6$ in Fraktion 1 und $s_{22}=0,4$ in Fraktion 2 bestimmt. Beide Fraktionen werden mittels DSC untersucht. Die überlagernden Kristallisationspeaks beider Polymere liefern eine Kristallisationsenthalpie von -260 mJ für die Fraktion 1 und eine Kristallisationsenthalpie von -248 mJ für Fraktion 2. Die Auswertung nach Gleichung 19 und 26 liefert die in der Ausgangsprobe enthaltenen Massen für P1 von 1,52 mg und für P2 von 3,02 mg.

Gleiches lässt sich auch für Proben mit mehr als zwei sich überlagernden thermischen Ereignissen von Polymeren durchführen. Für jedes weitere Polymer ist dann jeweils mindestens eine weitere Fraktion erforderlich. Eine Fraktion kann auch durch die Ausgangsprobe ersetzt werden. Weiterhin ist es möglich, durch die Bestimmung der Massenbilanz (Wägung der Fraktionen und der Ausgangsprobe) eine DSC-Messung zu ersetzten.

Die statistische Qualität des Ergebnisses kann durch eine höhere Anzahl an Fraktionen und Messungen erhöht werden. Die notwendige Anzahl an Fraktionen bei unbekannten Peaks kann entweder mit der DSC durch einen Datenbankabgleich für ein thermisches Ereignis oder durch Anwendung weiterer Analysemethoden wie Pyrolyse-GCMS, FTIR-Spektroskopie, IR-Mikroskopie, Raman-Spektroskopie oder Raman-Mikroskopie erfolgen. Beim Datenbankabgleich werden zunächst Vorschläge für Polymere erhalten, welche potentiell enthalten sein können. Durch die Untersuchung der entsprechenden Anzahl an Fraktionen würden in der Probe vorhandene Polymere mit einem Massenanteil >0 und nicht vorhandene Polymere mit einem Massenanteil gleich 0 ausgewiesen werden.

Grundsätzlich ist das erfindungsgemäße Verfahren nicht nur auf die Fraktionen aus der Elektroseparation anwendbar, sondern kann mit jeder Kombination aus einer Fraktioniermethode, die bei gegebenen Separationsparametern zu einer in Abhängigkeit von der Ausgangszusammensetzung definierten Zusammensetzung in der Fraktion führt und einer Analysenmethode, die eine Quantifizierung der Inhaltstoffe ermöglicht, angewendet werden, wenn zwei oder mehr Inhaltstoffe überlagernde Messsignale verursachen.

Vorrichtungen zur Durchführung der Elektroseparation ergeben sich aus der nachfolgenden Beschreibung mit Bezugnahme auf die zugehörigen Zeichnungen. Es zeigen:

Figur 1:  eine schematische Darstellung einer Vorrichtung zur Durchführung einer Elektroseparation einer Polymerpartikel enthaltenden Probe und

Figur 2:  eine schematische Darstellung eines triboelektrischen Freifallabscheiders zur Elektroseparation einer Polymerpartikel enthaltenden Probe.

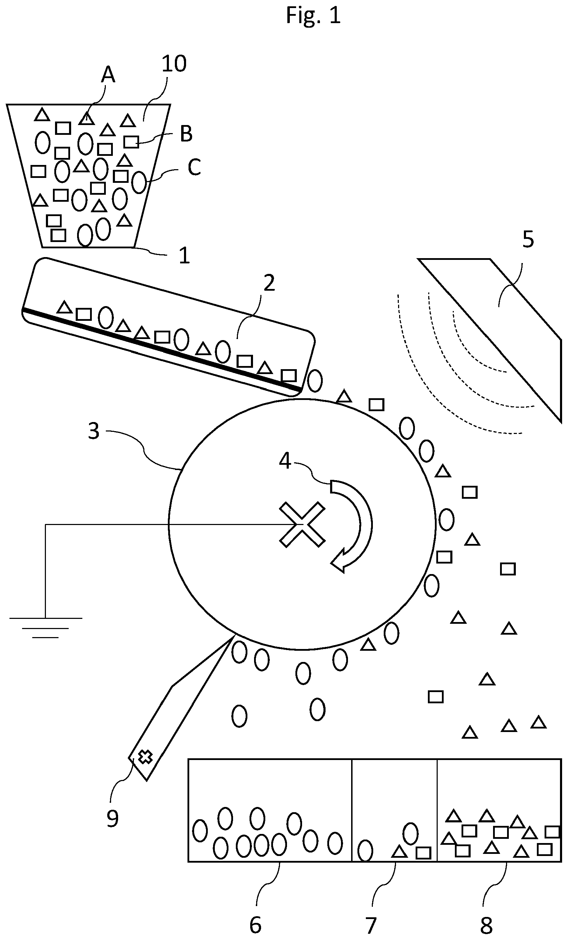

**Figur 1** zeigt eine schematische Darstellung einer Vorrichtung zur Durchführung einer Elektroseparation einer Polymerpartikel enthaltenden Probe 10. Die Vorrichtung weist einen Probenvorlagebehälter 1 auf, in welchem eine Polymerpartikel enthaltende Probe 10, wobei es sich um eine Sedimentprobe handeln kann, enthalten ist. Ferner weist die Vorrichtung eine Vibrationsrinne 2 und eine sich an die Vibrationsrinne 2 anschließende Metalltrommel 3 auf, welche elektrisch geerdet ist. Das Ende der Vibrationsrinne 2 ist derart angeordnet, dass Probenmaterial der Polymerpartikel enthaltenden Probe 10 von der Vibrationsrinne 2 auf die Oberfläche der Metalltrommel 3 gelangen kann. Der Pfeil 4 kennzeichnet die Rotationsrichtung der Metalltrommel 3.

Weiterhin weist die Vorrichtung einen Hochvoltgenerator auf, welcher mit einer Koronaelektrode 5 elektrisch kontaktiert ist. Die Koronaelektrode 5 ist in einem Abstand von der Oberfläche der Metalltrommel 3 so angeordnet, dass das entlang der Oberfläche der Metalltrommel 3 geführte Probenmaterial der Polymerpartikel enthaltenden Probe 10 in den Einflussbereich der Koronaelektrode 5 gelangt. In einem Abstand von der Metalltrommel 3 sind drei Auffangbehälter 6, 7 und 8 nebeneinander angeordnet. Weiterhin ist in einem vorgegebenen Abstand von der Oberfläche der Metalltrommel 3 ein Prellblech 9 angeordnet, um an der Metalltrommeloberfläche anhaftende Partikel der Polymerpartikel enthaltenden Probe 10 abzustreifen.

Die Polymerpartikel enthaltende Probe 10 enthält Partikel A, welche als Dreiecke dargestellt sind, Partikel B, welche als Rechtecke dargestellt sind, sowie Partikel C, welche oval dargestellt sind. Die Partikel A, B und C gelangen aus dem Probenvorlagebehälter 1 in die Vibrationsrinne 2, wobei die Partikel A, B und C aufgrund von Stößen vereinzelt werden. Anschließend gelangen die Partikel A, B und C auf die Oberfläche der Metalltrommel 3, wobei die Partikel A, B und C aufgrund der Rotation der Metalltrommel 3 in den Einflussbereich der Koronaelektrode 5 gelangen. Durch den Einfluss der Koronaelektrode 5 werden die Polymerpartikel A, B und C an der Metalltrommel 3 unterschiedlich stark entladen und dementsprechend von der Oberfläche der Metalltrommel 3 gelöst.

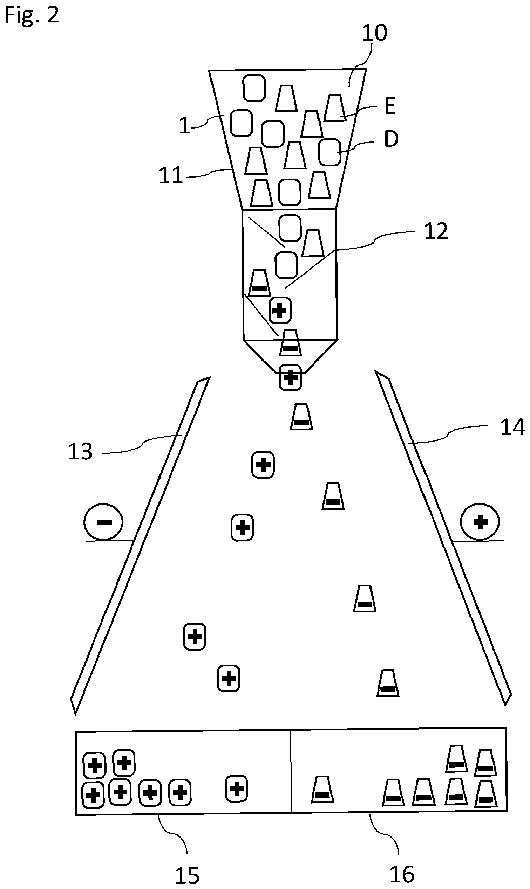

Leitende und/oder halbleitende Partikel A und B, wie beispielsweise Metallpartikel und Sandpartikel gelangen dabei in die Auffangbehälter 7 und 8, wobei nichtleitende Partikel C, wie Polymere, am längsten an der Metalltrommel 3 haften und von dem Prellblech 9 abgestreift werden, wodurch die Partikel C in den Auffangbehälter 6 gelangen. Dabei können die Partikel C Partikel unterschiedlicher Polymerspezies umfassen. Die im Auffangbehälter 6 angereicherten Partikel C können einer weiteren Elektroseparation unterzogen werden, um die enthaltenen Polymerspezies zu quantifizieren. Die weitere Elektroseparation kann mit einem triboelektrischen Freifallabscheider erfolgen, wie er in Figur 2 gezeigt ist. Die in Figur 1 beschriebene Vorrichtung dient im Wesentlichen zur Vorseparation beziehungsweise Probenvorbereitung im Rahmen der Probenbereitstellung zur Durchführung des erfindungsgemäßen Verfahrens.

Die **Figur 2** zeigt eine schematische Darstellung eines triboelektrischen Freifallabscheiders zur Elektroseparation einer Polymerpartikel enthaltenden Probe 10. Der triboelektrische Freifallabscheider weist eine Probenvorlage 11 auf, in welcher die Probe 10 enthalten ist. Bei der Probe 10 kann es sich um eine mit der in Figur 1 gezeigten Vorrichtung vorseparierte Probe handeln. Weiterhin weist der triboelektrische Freifallabscheider eine triboelektrische Aufladeeinheit 12 auf, in welche aus der Probenvorlage 11 Partikel D und E gelangen, wobei die Partikel D und E triboelektrisch aufgeladen werden. Nach der triboelektrischen Aufladeeinheit 12 sind Elektroden 13 und 14 so angeordnet, dass zwischen ihnen ein elektrisches Feld ausgebildet ist. Die triboelektisch aufgeladenen Partikel D und E fallen aus der triboelektrischen Aufladeeinheit 12 in das zwischen den Elektroden 13 und 14 ausgebildete elektrische Feld, wobei der freie Fall der aufgeladenen Partikel D und E entsprechend ihrer Ladung abgelenkt wird. Infolge der Ablenkung im elektrischen Feld werden die Partikel D und E in nebeneinander angeordnete Auffangbehälter 15 und 16 separiert.

Eine Bestimmung der polymerspezifischen Anreicherungsfaktoren kann mit der in Figur 1 gezeigten Vorrichtung und mit dem in Figur 2 gezeigten triboelektrischen Freifallabscheider durchgeführt werden.

**Bezugszeichenliste**

| | |
|---|---|
| 1 | Probenvorlagebehälter |
| 2 | Vibrationsrinne |
| 3 | Metalltrommel |
| 4 | Rotationsrichtung |
| 5 | Koronaelektrode |
| 6 | Auffangbehälter |
| 7 | Auffangbehälter |
| 8 | Auffangbehälter |
| 9 | Prellblech |
| 10 | Polymerpartikel enthaltene Probe |
| 11 | Probenvorlage |
| 12 | triboelektrische Aufladeeinheit |
| 13 | Elektrode |
| 14 | Elektrode |
| 15 | Auffangbehälter |
| 16 | Auffangbehälter |
| A, B, C, D, E | Partikel |

**Patentansprüche**

1. Verfahren zur Quantifizierung von Polymerspezies in einer Polymerpartikel enthaltenden Probe, bei welchem

a) polymerspezifische Anreicherungsfaktoren bestimmt werden, in dem für verschiedene reine Polymerspezies eine Elektroseparation durchgeführt wird, wobei Polymerspeziespartikel einer vorgegebenen Partikelgröße oder eines vorgegebenen Partikelgrößenbereichs bei vorgegeben Separationsparametern elektrisch aufgeladen und elektrostatisch in nebeneinander angeordneten Auffangbehältern (6, 7, 8, 15, 16) getrennt werden, wobei für jeden Auffangbehälter (6, 7, 8, 15, 16) ein polymerspezifischer Anreicherungsfaktor des in dem jeweiligen Auffangbehälter (6, 7, 8, 15, 16) enthaltenden Anteils der getrennten Polymerspezies bestimmt wird, oder

b) polymerspezifische, separationsparameterabhängige Anreicherungsfaktoren von verschiedenen reinen Polymerspezies von einer Datenbank bereitgestellt werden,

c) eine Polymerpartikel enthaltende Probe (10) bereitgestellt wird, deren Partikel eine Größe aufweisen, welche der vorgegebenen Partikelgröße oder dem vorgegebenen Partikelgrößenbereich entspricht,

d) eine Elektroseparation der Polymerpartikel enthaltenden Probe (10) bei den vorgegebenen Separationsparametern durchgeführt wird, wobei die Polymerpartikel der Polymerpartikel enthaltenden Probe (10) elektrisch aufgeladen und elektrostatisch in die nebeneinander angeordneten Auffangbehälter (6, 7, 8, 15, 16) getrennt werden,

e) in jedem der vorgegebenen Auffangbehälter (6, 7, 8, 15, 16) eine spezifische Enthalpie und/oder eine spezifische Wärmekapazitätsänderung der in dem jeweiligen Anreicherungsbehälter angereicherten Polymerpartikel der Polymerpartikel enthaltenden Probe (10) bestimmt wird und

f) aus den bestimmten spezifischen Enthalpien und/oder den bestimmten spezifischen Wärmekapazitätsänderungen und den polymerspezifischen Anreicherungsfaktoren die Massenanteile der in der Polymerpartikel enthaltenden Probe (10) enthaltenden Polymerspezies bestimmt werden.

2. Verfahren nach Anspruch 1, **dadurch gekennzeichnet, dass** vor der Elektroseparation der Polymerpartikel enthaltenden Probe (10) eine mittlere Partikelgröße der Polymerpartikel enthaltenden Probe (10) bestimmt wird, wobei die mittlere Partikelgröße bei der Bestimmung der polymerspezifischen Anreicherungsfaktoren berücksichtigt wird.

3. Verfahren nach Anspruch 1 oder 2, **dadurch gekennzeichnet, dass** eine Vorbehandlung der Polymerpartikel enthaltenden Probe (10) vorgesehen wird, bei welcher Partikel bereitgestellt werden, welche eine Größe von 1 mm nicht überschreiten.

4. Verfahren nach einem der Ansprüche 1 bis 3, **dadurch gekennzeichnet, dass** eine Bestimmung des polymerspezifischen Anreicherungsfaktors für Polymerspeziesproben reiner Polymerspezies durchgeführt werden, welche aus Partikeln mit einer Größe im Bereich von 5 mm bis 1 mm, 1 mm bis 0,1 mm, 0,1 mm bis 0,01 mm, 0,01 mm bis 0,001 mm bestehen, wobei eine zu quantifizierende Polymerpartikel enthaltende Probe (10) derart vorbehandelt wird, dass die zu quantifizierende Polymerpartikel enthaltende Probe aus Partikeln mit einer Größe im Bereich von 5 mm bis 1 mm, 1 mm bis 0,1 mm, 0,1 mm bis 0,01 mm oder 0,01 mm bis 0,001 mm besteht.

5. Verfahren nach einem der Ansprüche 1 bis 4, **dadurch gekennzeichnet, dass** die Polymerpartikel enthaltende Probe (10) und/oder reine Polymerspeziespartikel zum Erreichen des vorgegebenen Partikelgrößenbereichs oder der vorgegebenen Partikelgröße gemahlen, vorzugsweise kryogemahlen, wird/werden.

6. Verfahren nach einem der Ansprüche 1 bis 5, **dadurch gekennzeichnet, dass** bei der Probenbereitstellung der Polymerpartikel enthaltenden Probe (10) nicht-aufladbare Bestandteile abgetrennt werden.

7. Verfahren nach einem der Ansprüche 1 bis 6, **dadurch gekennzeichnet, dass** bei der elektrostatischen Trennung mindestens drei Auffangbehälter (6, 7, 8, 15, 16) vorgegeben werden.

8. Verfahren nach einem der Ansprüche 1 bis 7, **dadurch gekennzeichnet, dass** vor der Elektroseparation der Polymerpartikel enthaltenden Probe (10) eine Anzahl der in der Polymerpartikel enthaltenden Probe (10) enthaltenen verschiedenen Polymerspezies bestimmt wird.

9. Verfahren nach Anspruch 8, **dadurch gekennzeichnet, dass** die Anzahl der Auffangbehälter (6, 7, 8, 15, 16) in Schritt a) und Schritt d) entsprechend der Anzahl der in der Polymerpartikel enthaltenden Probe (10) enthaltenen Polymerspezies festgelegt wird.

10. Verfahren nach einem der Ansprüche 1 bis 9, **dadurch gekennzeichnet, dass** zumindest eine in einem Auffangbehälter (6, 7, 8, 15, 16) angereicherte Fraktion der Polymerpartikel enthaltenden Probe einer weiteren Elektroseparation mit geänderten Separationsparametern unterzogen wird.

11. Verfahren nach einem der Ansprüche 1 bis 10, **dadurch gekennzeichnet, dass** die Separationsparameter polymerspezifisch angepasst werden.

12. Verfahren nach einem der Ansprüche 1 bis 11, **dadurch gekennzeichnet, dass** als Separationsparameter eine Feuchte der Polymerpartikel enthaltenden Probe (10), eine elektrische Spannung, eine elektrische Feldstärke und/oder eine Partikelgeschwindigkeit berücksichtigt wird/werden.

13. Verfahren nach einem der Ansprüche 1 bis 12, **dadurch gekennzeichnet, dass** die spezifische Enthalpie und/oder eine spezifische Wärmekapazitätsänderung mittels Differenzkalorimetrie bestimmt wird.

**Claims**

1. A method for quantifying polymer species in a sample containing polymer particles, wherein

a) polymer-specific enrichment factors are determined by carrying out an electro-separation for different pure polymer species, wherein polymer species particles of a predefined particle size or a predefined particle size range at predefined separation parameters are electrically charged and electrostatically separated in collection containers (6, 7, 8, 15, 16) arranged next to one another, wherein a polymer-specific enrichment factor of the portion of the separated polymer species contained in the respective collection container (6, 7, 8, 15, 16) is determined for each collection container (6, 7, 8, 15, 16), or

b) polymer-specific enrichment factors from different pure polymer species and depending on the separation parameters are provided from a database,

c) a sample (10) containing polymer particles is provided, wherein the particles have a size which corresponds to the predefined particle size or the predefined particle size range,

d) an electro-separation of the sample (10) containing polymer particles is carried out with the predefined separation parameters, wherein the polymer particles of the sample (10) containing polymer particles are electrically charged and electrostatically separated in the collection containers (6, 7, 8, 15, 16) arranged next to one another,

e) a specific enthalpy and/or a specific heat capacity change of the polymer particles of the sample (10) containing polymer particles enriched in the respective enrichment container is determined in each one of the predefined collection containers (6, 7, 8, 15, 16), and

f) the mass percentages of the polymer species contained in the sample (10) containing polymer particles are determined from the determined specific enthalpies and/or the determined specific heat capacity changes and the polymer-specific enrichment factors.

2. The method according to claim 1, **characterized in that** a mean particle size of the sample (10) containing polymer particles is determined before the electro-separation of the sample (10) containing polymer particles, wherein the mean particle size is considered when determining the polymer-specific enrichment factors.

3. The method according to claim 1 or 2, **characterized in that** a pre-treatment of the sample (10) containing polymer particles is envisaged, wherein particles are provided which are not larger than 1 mm.

4. The method according to any one of claims 1 to 3, **characterized in that** a determination of the polymer-specific enrichment factor for polymer species samples of pure polymer species which consist of particles with a size in the range from 5 mm to 1 mm, 1 mm to 0.1 mm, 0.1 mm to 0.01 mm, 0.01 mm to 0.001 mm is carried out, wherein a sample (10) containing polymer particles to be quantified is pre-treated such that the sample containing polymer particles to be quantified consists of particles with a size in the range from 5 mm to 1 mm, 1 mm to 0.1 mm, 0.1 mm to 0.01 mm or 0.01 mm to 0.001 mm.

5. The method according to any one of claims 1 to 4, **characterized in that** the sample (10) containing polymer particles and/or pure polymer species particles is/are grinded, preferably cryo-grinded, in order to reach the predefined particle size range or the predefined particle size.

6. The method according to any one of claims 1 to 5, **characterized in that** non-chargeable components are separated when providing the sample (10) containing polymer particles.

7. The method according to any one of claims 1 to 6, **characterized in that** at least three collection containers (6, 7, 8, 15, 16) are predefined for the electrostatic separation.

8. The method according to any one of claims 1 to 7, **characterized in that** a number of the different polymer species contained in the sample (10) containing polymer particles is determined before the electro-separation of the sample (10) containing polymer particles.

9. The method according to claim 8, **characterized in that** the number of the collection containers (6, 7, 8, 15, 16) is set in step a) and step d) corresponding to the number of the polymer species contained in the sample (10) containing polymer particles.

10. The method according to any one of claims 1 to 9, **characterized in that** at least a fraction of the sample containing polymer particles enriched in a collection container (6, 7, 8, 15, 16) is subjected to a further electro-separation with changed separation parameters.

11. The method according to any one of claims 1 to 10, **characterized in that** the separation parameters are adapted in a polymer-specific manner.

**12.** The method according to any one of claims 1 to 11, **characterized in that** a moisture of the sample (10) containing polymer particles, an electric voltage, an electric field strength and/or a particle velocity is/are considered as separation parameters.

**13.** The method according to any one of claims 1 to 12, **characterized in that** the specific enthalpy and/or a specific heat capacity change is determined by means of differential calorimetry.

**Revendications**

**1.** Procédé de quantification d'espèces polymères dans un échantillon contenant des particules polymères, selon lequel

a) des facteurs d'enrichissement spécifiques aux polymères sont déterminés en réalisant une séparation électrique pour différentes espèces polymères pures, les particules d'espèces polymères d'une taille de particule prédéfinie ou d'une plage de taille de particule prédéfinie étant chargées électriquement avec des paramètres de séparation prédéfinis et étant séparées par procédé électrostatique dans des récipients collecteurs (6, 7, 8, 15, 16) disposés les uns à côté des autres, un facteur d'enrichissement spécifique aux polymères de la part des espèces polymères séparées contenues dans le récipient collecteur respectif (6, 7, 8, 15, 16) étant déterminé pour chaque récipient collecteur (6, 7, 8, 15 ,16), ou
b) des facteurs d'enrichissement de différentes espèces polymères pures, spécifiques aux polymères et dépendants des paramètres de séparation sont fournis par une base de données,
c) un échantillon (10) contenant des particules polymères est fourni dont les particules présentent une taille qui correspond à la taille de particule ou à la plage de taille prédéfinie,
d) une séparation électrique de l'échantillon (10) contenant des particules polymères est réalisée avec les paramètres de séparation prédéfinis, les particules polymères de l'échantillon (10) contenant des particules polymères étant chargées électriquement et séparées par procédé électrostatique dans les récipients collecteurs (6, 7, 8, 15, 16) disposés les uns à côté des autres,
e) une enthalpie spécifique et/ou une modification spécifique de la capacité thermique des particules polymères enrichies dans le récipient collecteur respectif de l'échantillon (10) contenant des particules polymères est déterminée dans chacun des récipients collecteurs prédéfinis (6, 7, 8, 15, 16), et
f) les fractions massiques des espèces polymères contenues dans l'échantillon (10) contenant les particules polymères sont déterminées à partir des enthalpies spécifiques déterminées et/ou des modifications spécifiques déterminées de la capacité thermique et des facteurs d'enrichissement spécifiques aux polymères.

**2.** Procédé selon la revendication 1, **caractérisé en ce qu'**avant la séparation électrique de l'échantillon (10) contenant les particules polymères, une taille de particule moyenne de l'échantillon (10) contenant des particules polymères est déterminée, la taille de particule moyenne étant prise en compte lors de la détermination des facteurs d'enrichissement spécifiques aux polymères.

**3.** Procédé selon la revendication 1 ou 2, **caractérisé en ce qu'**un prétraitement de l'échantillon (10) contenant des particules polymères est prévu lors duquel des particules dont la taille ne dépasse pas 1 mm sont mises à disposition.

**4.** Procédé selon l'une quelconque des revendications 1 à 3, **caractérisé en ce qu'**une détermination du facteur d'enrichissement spécifique aux polymères est réalisée pour des échantillons d'espèces de polymères pures constitués de particules d'une taille dans la plage de 5 mm à 1 mm, 1 mm à 0,1 mm, 0,1 mm à 0,01 mm, 0,01 mm à 0,001 mm, un échantillon (10) contenant des particules polymères à quantifier étant prétraité de sorte que l'échantillon contenant des particules polymères à quantifier est constitué de particules d'une taille dans la plage de 5 mm à 1 mm, 1 mm à 0,1 mm, 0,1 mm à 0,01 mm ou 0,01 mm à 0,001 mm.

**5.** Procédé selon l'une quelconque des revendications 1 à 4, **caractérisé en ce que** l'échantillon (10) contenant des particules polymères et/ou des particules d'espèces polymères pures sont broyées, de préférence cryobroyées, pour atteindre la plage de taille de particules prédéfinie ou la taille de particules prédéfinie.

**6.** Procédé selon l'une quelconque des revendications 1 à 5, **caractérisé en ce que** lors de la mise à disposition de l'échantillon (10) contenant des particules polymères, des composants non rechargeables sont séparés.

**7.** Procédé selon l'une quelconque des revendications 1 à 6, **caractérisé en ce que** lors de la séparation électrostatique, au moins trois récipients collecteurs (6, 7, 8, 15, 16) sont prédéfinis.

**8.** Procédé selon l'une quelconque des revendications 1 à 7, **caractérisé en ce qu'**avant la séparation électrique de l'échantillon (10) contenant des particules polymères, un nombre des différentes espèces polymères contenues dans l'échantillon (10) contenant des particules polymères est déterminé.

**9.** Procédé selon la revendication 8, **caractérisé en ce que** le nombre des récipients collecteurs (6, 7, 8, 15, 16) est déterminé à l'étape a) et l'étape d) selon le nombre d'espèces polymères contenues dans l'échantillon (10) contenant des particules polymères.

**10.** Procédé selon l'une quelconque des revendications 1 à 9, **caractérisé en ce qu'**au moins une fraction enrichie dans un récipient collecteur (6, 7, 8, 15, 16) de l'échantillon contenant des particules polymères est soumise à une séparation électrique supplémentaire avec des paramètres de séparation modifiés.

**11.** Procédé selon l'une quelconque des revendications 1 à 10, **caractérisé en ce que** les paramètres de séparation sont adaptés spécifiquement aux polymères.

**12.** Procédé selon l'une quelconque des revendications 1 à 11, **caractérisé en ce qu'**une humidité de l'échantillon (10) contenant des particules polymères, une tension électrique, une force de champ électrique et/ou une vitesse de particules est/sont pris en compte comme paramètres de séparation.

**13.** Procédé selon l'une quelconque des revendications 1 à 12, **caractérisé en ce que** l'enthalpie spécifique et/ou une modification spécifique de la capacité thermique sont déterminées au moyen d'une analyse calorimétrique différentielle.

# Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012003935 A1

- DE 19901743 A1

**In der Beschreibung aufgeführte Nicht-Patentliteratur**

- **LAURA CUTRONEO et al.** Microplastics in seawater: sampling strategies, laboratory methodologies, and identification techniques applied to port environment. *Environmental Science and Polution Research,* 06. Februar 2020, vol. 27 (9), ISSN 0944-1344, 8938-8952

- **BIN ZHANG et al.** Research Progress of Microplastics in Freshwater Sediments in China. *Environmental Science and Polution Research,* 08. Juni 2020, vol. 27 (25), ISSN 0944-1344, 31046-31060